# EUROPEAN PATENT APPLICATION

(11) **EP 1 889 841 A1**
(43) Date of publication of application: **20.02.2008**
(21) Application number: 06757024.2
(22) Date of filing: 06.06.2006
(51) Int. Cl.: C07D 401/12, A61K 9/14, A61K 9/48, A61K 31/4439, A61P 1/04, A61P 31/04

(54) **CRYSTAL OF SALT OF BENZIMIDAZOLE COMPOUND**

(30) Priority: 07.06.2005 JP 2005166972
(71) Applicant: Takeda Pharmaceutical Company Limited, Osaka-shi, Osaka 541-0045 (JP)
(72) Inventor: HASHIMOTO, Hideo TAKEDA PHARMACEUTICAL COMPANY LTD, Osaka-shi, Osaka 5328686 (JP); URAI, Tadashi TAKEDA PHARMACEUTICAL COMPANY LTD, Osaka-shi, Osaka 5328686 (JP)
(74) Representative: Wright, Robert Gordon McRae
(86) International application number: PCT/JP2006/311284
(87) International publication number: WO 2006/132217

(57) **Abstract**

A crystal of a salt of 2-[[[3-methyl-4-(2,2,2-trifluoroethoxy)-2-pyridinyl]methyl]sulfinyl]-1H-benzimidazole which is useful as a medicine, and use of the crystal.

## Description

### Technical Field

The present invention relates to crystal of a salt of a benzimidazole compound having an antiulcer action or the like.

### Background Art

2-[[[3-Methyl-4-(2,2,2-trifluoroethoxy)-2-pyridinyl]methyl]sulfinyl]-1H-benzimidazole (lansoprazole) or a salt thereof which has an antiulcer activity has been described in Patent document 1 and the like.
Patent Document 1: JP-A 61-50978

### Disclosure of the invention

### Problems to be solved by the invention

A crystal of 2-[[[3-methyl-4-(2,2,2-trifluoroethoxy)-2-pyridinyl]methyl]sulfinyl]-1H-benzimidazole (free form) which is low toxic and excellent in safety as a medicine has been used as a useful medicine in the medical field. However, a crystal of a salt of 2-[[[3-methyl-4-(2,2,2-trifluoroethoxy)-2-pyridinyl]methyl]sulfinyl]-1H-benzimidazole which is more stable and more easily handled than the free form thereof which has been already practically used has never been known and obtained.

### Means for Solving the Problem

As a result of intensive investigations, the present inventors have succeeded in crystallization of a salt of 2-[[[3-methyl-4-(2,2,2-trifluoroethoxy)-2-pyridinyl]methyl]sulfinyl]-1H-benzimidazole, and have found that the crystal has unexpectedly excellent stability and the like, thereby the present invention has been completed.

That is, the present invention provides:
(1) a crystal of a salt of 2-[[[3-methyl-4-(2,2,2-trifluoroethoxy)-2-pyridinyl]methyl]sulfinyl]-1H-benzimidazole;
(2) the crystal according to the above (1), which is a crystal of a sodium salt of 2-[[[3-methyl-4-(2,2,2-trifluoroethoxy)-2-pyridinyl]methyl]sulfinyl]-1H-benzimidazole;
(3) the crystal according to the above (2), which has infrared absorption spectra at wavenumbers of about 1584, 1266, 1182, 1025 and 742 cm⁻¹;
(4) the crystal according to the above (2), which has an X-ray powder diffraction pattern whose characteristic peaks appear at about 5.64, 14.24, and 20.96° that are diffraction angles represented by 2θ in X-ray powder diffraction;
(5) a pharmaceutical composition comprising the crystal according to the above (1);
(6) the pharmaceutical composition according to the above (5), which is a prophylactic or remedy for peptic ulcer, Zollinger-Ellison syndrome, gastritis, reflux esophagitis, symptomatic GERD, NUD (Non-Ulcer Dyspepsia), gastric cancer, gastric MALT lymphoma or hyperacidity, an anti-Helicobacter pylori agent, or an inhibitor for upper gastrointestinal hemorrhage due to peptic ulcer, acute stress ulcer, hemorrhagic gastritis or invasive stress;
(7) a method for preventing or treating peptic ulcer, Zollinger-Ellison syndrome, gastritis, reflux esophagitis, symptomatic GERD, NUD, gastric cancer, gastric MALT lymphoma or hyperacidity, eliminating Helicobacter pylori, or inhibiting upper gastrointestinal hemorrhage due to peptic ulcer, acute stress ulcer, hemorrhagic gastritis or invasive stress, which comprises administering an effective amount of the crystal of the above (1) to a mammal;
(8) use of the crystal according to the above (1) for production of a prophylactic or remedy for peptic ulcer, Zollinger-Ellison syndrome, gastritis, reflux esophagitis, symptomatic GERD, NUD, gastric cancer, gastric MALT lymphoma or hyperacidity, an anti-Helicobacter pylori agent, or an inhibitor for upper gastrointestinal hemorrhage due to peptic ulcer, acute stress ulcer, hemorrhagic gastritis or invasive stress; and the like.

### Effect of the invention

The crystal of a salt of 2-[[[3-methyl-4-(2,2,2-trifluoroethoxy)-2-pyridinyl]methyl]sulfinyl]-1H-benzimidazole of the present invention (hereinafter, sometimes, referred to as "the crystal of the present invention") is useful as a medicine because the crystal of the present invention has low toxicity and has an excellent antiulcer activity, gastric acid antisecretory activity, mucosal protective activity, anti-Helicobacter pylori activity and the like. Crystallization of a salt of 2-[[[3-methyl-4-(2,2,2-trifluoroethoxy)-2-pyridinyl]methyl]sulfinyl]-1H-benzimidazole provides not only improvement in stability of said salt but also facilitation of handling of said salt. Thus, using the crystal of the present invention, a solid pharmaceutical composition can be produced with good reproducibility.

The improved stability makes the crystal of the present invention less sensitive to humidity, so that a solid pharmaceutical composition comprising the crystal of the present invention can be stored at normal temperature for a long time, for example, even without a desiccating agent.

The crystal of the present invention has higher solubility and more rapidly dissolves than the free form. Therefore, for example, when the crystal of the present invention is formulated into an injectable preparation, the time required to dissolve or dilute an injectable preparation in a solvent or with a diluent prior to use is shortened and the injectable preparation is also easy to handle.

When the crystal of the present invention is formulated into an oral preparation, the crystal of the present invention is absorbed well and rapidly exerts its effect, so that an excellent pharmaceutical preparation can be provided.

### Brief Description of Drawings

Fig. 1 is an example of an infrared absorption spectrum chart of a (amorphous) sodium salt of lansoprazole obtained in Reference Example 1.
Fig. 2 is an example of an X-ray powder diffraction chart of a (amorphous) sodium salt of lansoprazole obtained in Reference Example 1.
Fig. 3 is an example of an infrared absorption spectrum chart of a crystal of a sodium salt of lansoprazole obtained in Example 1.
Fig. 4 is an example of an X-ray powder diffraction chart of a crystal of a sodium salt of lansoprazole obtained in Example 1.

### Best Mode for Carrying Out the Invention

Examples of the "salt" of a salt of 2-[[[3-methyl-4-(2,2,2-trifluoroethoxy)-2-pyridinyl]methyl]sulfinyl]-1H-benzimidazole include a metal salt, a salt with an organic base, a salt with a basic amino acid and the like, and preferred is a physiologically acceptable salt.

Examples of the metal salt include an alkali metal salt such as a sodium salt and a potassium salt; an alkaline earth metal salt such as a calcium salt, a magnesium salt and a barium salt; and the like. Examples of the salt with an organic base include salts with trimethylamine, triethylamine, pyridine, picoline, ethanolamine, diethanolamine, triethanolamine, dicyclohexylamine, N,N-dibenzylethylenediamine and the like. Examples of the salt with a basic amino acid include salts with arginine, lysine and the like. Particularly preferred is a sodium salt.

The crystal of a salt of 2-[[[3-methyl-4-(2,2,2-trifluoroethoxy)-2-pyridinyl]methyl]sulfinyl]-1H-benzimidazole may be a hydrate or a non-hydrate. The crystal of a salt of 2-[[[3-methyl-4-(2,2,2-trifluoroethoxy)-2-pyridinyl]methyl]sulfinyl]-1H-benzimidazole may be a solvate or a non-solvate.

The crystal of a salt of 2-[[[3-methyl-4-(2,2,2-trifluoroethoxy)-2-pyridinyl]methyl]sulfinyl]-1H-benzimidazole can be obtained by addition of a base serving as a counterion to 2-[[[3-methyl-4-(2,2,2-trifluoroethoxy)-2-pyridinyl]methyl]sulfinyl]-1H-benzimidazole and then crystallization, or by crystallization of an amorphous salt of 2-[[[3-methyl-4-(2,2,2-trifluoroethoxy)-2-pyridinyl]methyl]thio]-1H-benzimidazole.

Examples of the "base" include an inorganic base, an organic base, a basic amino acid, and the like. Examples of the inorganic base include an alkali metal carbonate such as potassium carbonate and sodium carbonate, an alkali metal hydroxide such as sodium hydroxide and potassium hydroxide, an alkali metal hydride such as sodium hydride and potassium hydride, and the like. Examples of the organic base include an alkali metal alkoxide such as sodium methoxide and sodium ethoxide, an alkali metal carboxylate such as sodium acetate, amines such as piperidine, piperazine, pyrrolidine, morpholine, diethylamine, diisopropylethylamine and triethylamine, and pyridines such as pyridine and dimethylaminopyridine.

A salt of 2-[[[3-methyl-4-(2,2,2-trifluoroethoxy)-2-pyridinyl]methyl]sulfinyl]-1H-benzimidazole can be produced according to a method described in JP-A 61-50978, USP 4,628,098 or the like or a similar method thereto.

Examples of a method for crystallization include crystallization from a solution, crystallization from a vapor, and crystallization from a melt.

Examples of the "crystallization from a solution" include a concentration method, a slow cooling method, a reaction method (a diffusion method, or an electrolysis method), a hydrothermal growth method, a fusing agent method, and the like. Examples of a solvent to be used include aromatic hydrocarbons (e.g., benzene, toluene, xylene, etc.), halogenated hydrocarbons (e.g., dichloromethane, chloroform, etc.), saturated hydrocarbons (e.g., hexane, heptane, cyclohexane, etc.), ethers (e.g., diethyl ether, diisopropyl ether, tetrahydrofuran, dioxane, etc.), nitriles (e.g., acetonitrile, etc.), ketones (e.g., acetone etc.), sulfoxides (e.g., dimethylsulfoxide, etc.), acid amides (e.g., N,N-dimethylformamide etc.), esters (e.g., ethyl acetate etc.), alcohols (e.g., methanol, ethanol, isopropyl alcohol, etc.), water, and the like. These solvents may be used alone or as a mixture of two or more solvents at an appropriate ratio (e.g., 1:1 to 1:100). Particularly preferable solvents are ketones (e.g., acetone etc.) and esters (e.g., ethyl acetate etc.).

Examples of the "crystallization from a vapor" include an evaporation method (a sealed tube method or an air stream method), a vapor phase reaction method, a chemical transportation method and the like.

Examples of the "crystallization from a melt" include a normal freezing method (a pulling-up method, a temperature gradient method or a Bridgman method), a zone melting method (a zone leveling method or a float zone method), a special growth method (a VLS method or a liquid-phase epitaxy method) and the like.

General methods for analyzing the resulting crystal include a crystal analysis method by X-ray diffraction as well as analysis methods including a melting point measurement, infrared absorption (IR) and atomic absorption.

When the crystal of the present invention thus obtained is, for example, a crystal of a sodium salt, infrared absorption spectra appear at wavenumbers of about 1584, 1266, 1182, 1025 and 742 cm⁻¹, for example, at 1584 ± 8, 1266 ± 8, 1182 ± 8, 1025 ± 8 and 742 ± 8 cm⁻¹. The free form has a characteristic peak at 3240 cm⁻¹ due to stretching vibration of NH. However, the sodium salt of the present invention does not have such a peak.

The crystal of the sodium salt of the present invention has an X-ray powder diffraction pattern whose characteristic peaks appear in the vicinity of 5.64, 14.24, and 20.96°, for example at 5.64 ± 0.2, 14.24 ± 0.2, 17.18 ± 0.2, 19.72 ± 0.2, 20.50 ± 0.2, 20.96 ± 0.2, 21.40 ± 0.2, 29.14 ± 0.2°, which are diffraction angles represented by 2θ in X-ray powder diffraction.

The present compound is also useful for the treatment and prevention of peptic ulcer (e.g., gastric ulcer, gastric ulcer due to postoperative stress, duodenal ulcer, stomal ulcer, ulcer caused by a nonsteroidal antiinflammatory agent, etc.); gastritis; erosive esophagitis and nonerosive esophagitis; reflux esophagitis such as erosive or nonerosive reflux esophagitis; symptomatic gastroesophageal reflux disease (symptomatic GERD) including gastroesophageal reflux disease without esophagitis, such as erosive or nonerosive gastroesophageal reflux disease; NUD (Non-Ulcer Dyspepsia); gastric cancer (including gastric cancer accompanied with an enhanced production of interleukin-1β due to genetic polymorphism of interleukin-1); gastric MALT lymphoma; Zollinger-Ellison syndrome; hyperacidity (for example, hyperacidity and ulcer due to postoperative stress); or upper gastrointestinal hemorrhage due to peptic ulcer, acute stress ulcer, hemorrhagic gastritis, or invasive stress (stress resulting from major surgery necessitating intensive management after surgery, or from cerebral vascular disorder, head trauma, multiple organ failure or extensive burn necessitating intensive treatment) ; for pre-anesthetic administration; for anti-Helicobacter pylori (including bacteria elimination and assistance of bacteria elimination) or the like in mammals (e.g., human, monkey, sheep, bovine, horse, dog, cat, rabbit, rat, mouse, etc.).

As used herein, sometimes, reflux esophagitis and symptomatic gastroesophageal reflux disease (symptomatic GERD) are collectively referred to as GERD.

Accordingly, the pharmaceutical composition of the present invention is useful as a prophylactic or remedy for peptic ulcer, Zollinger-Ellison syndrome, gastritis, reflux esophagitis, symptomatic GERD, NUD, gastric cancer, gastric MALT lymphoma or hyperacidity; as an anti-Helicobacter pylori agent (including a bacteria elimination aid); as an inhibitor for upper gastrointestinal hemorrhage due to peptic ulcer, acute stress ulcer and hemorrhagic gastritis or invasive stress, or the like.

The content of a crystal of a salt of 2-[[[3-methyl-4-(2,2,2-trifluoroethoxy)-2-pyridinyl]methyl]sulfinyl]-1H-benzimidazole in the pharmaceutical composition of the present invention is about 0.01 to 100% by weight relative to the whole composition. The dose of the pharmaceutical composition of the present invention varies depending on a subject of administration, a route of administration, a target disease etc., and for example, it is about 0.5 to about 1,500 mg/day, preferably about 5 to about 150 mg/day of the active ingredient, when the pharmaceutical composition of the present invention is orally administered as an antiulcer agent to an adult human (60 kg). The compound of the present invention may be administered once a day or in 2 to 3 divided doses per day.

The crystal of the present invention is low toxic. Thus, the crystal of the present invention can be safely administered orally or parenterally (e.g., topical, rectal, intravenous administration, etc.), as it is or as a pharmaceutical composition prepared by mixing the crystal of the present invention with a pharmacologically acceptable carrier according to a per se method, e.g. as a preparation such as a tablet (including a sugar-coated tablet and a film-coated tablet), a powder, a granule, a capsule (including a soft capsule), an orally disintegrating tablet, a liquid, an injectable preparation, a suppository, a sustained-release preparation or a patch. Particularly, the crystal of the present invention is preferably administered as an oral preparation such as a tablet, a granule or a capsule or as an injectable preparation.

The injectable preparation may be a liquid injectable preparation, or a solid injectable preparation such as a lyophilized injectable preparation or a powdery injectable preparation. The solid injectable preparation can be dissolved in or diluted with a solvent substantially free of a non-aqueous solvent.

Examples of a pharmacologically acceptable carrier that may be used in producing the pharmaceutical composition of the present invention include various organic and inorganic carrier substances that are conventionally used as a formulation material, for example, an excipient, a lubricant, a binder, a disintegrant, a water-soluble polymer and a basic inorganic salt for a solid preparation; and a solvent, a solubilizing agent, a suspending agent, a tonicity agent, a buffering agent and a soothing agent for a liquid preparation. A conventional additive such as a preservative, an antioxidant, a coloring agent, a sweetener, an acidifier, a foaming agent or a flavor may also be used if necessary.

Examples of the "excipient" include lactose, sucrose, D-mannitol, starch, cornstarch, crystalline cellulose, light silicic anhydride, titanium oxide and the like.

Examples of the "lubricant" include magnesium stearate, a sucrose fatty acid ester, polyethylene glycol, talc, stearic acid and the like.

Examples of the "binder" include hydroxypropyl cellulose, hydroxypropylmethyl cellulose, crystalline cellulose, starch, polyvinylpyrrolidone, gum arabic powder, gelatin, pullulan, low-substituted hydroxypropyl cellulose and the like.

Examples of the "disintegrant" include (1) crospovidone, (2) disintegrants called super-disintegrants such as croscarmellose sodium (FMC-Asahi Kasei), carmellose calcium (Gotoku Chemical Company Ltd.) and the like, (3) carboxymethyl starch sodium (e.g., product of Matsutani Chemical Industry Co., Ltd.), (4) low-substituted hydroxypropyl cellulose (e.g., product of Shin-Etsu Chemical Co., Ltd.), (5) cornstarch, and the like. The "crospovidone" may be any crosslinked polymer having the chemical name 1-ethenyl-2-pyrrolidinone homopolymer, including polyvinylpyrrolidone (PVPP) and 1-vinyl-2-pyrrolidinone homopolymer, and specific examples thereof include Kollidon CL (produced by BASF), Polyplasdone XL (produced by ISP), Polyplasdone XL-10 (produced by ISP), Polyplasdone INF-10 (produced by ISP) and the like.

Examples of the "water-soluble polymer" include ethanol-soluble water-soluble polymers [e.g., cellulose derivatives such as hydroxypropyl cellulose (hereinafter also referred to as HPC), polyvinylpyrrolidone, etc.], ethanol-insoluble water-soluble polymers [e.g., cellulose derivatives such as hydroxypropylmethyl cellulose (hereinafter also referred to as HPMC), methyl cellulose and carboxymethyl cellulose sodium, sodium polyacrylate, polyvinyl alcohol, sodium alginate, guar gum, etc.] and the like.

Examples of the "basic inorganic salt" include basic inorganic salts of sodium, potassium, magnesium and/or calcium. Preferred are basic inorganic salts of magnesium and/or calcium. More preferred are basic inorganic salts of magnesium. Examples of basic inorganic salts of sodium include sodium carbonate, sodium hydrogen carbonate, disodium hydrogen phosphate, and the like. Examples of basic inorganic salts of potassium include potassium carbonate, potassium hydrogen carbonate, and the like. Examples of basic inorganic salts of magnesium include heavy magnesium carbonate, magnesium carbonate, magnesium oxide, magnesium hydroxide, magnesium metasilicate aluminate, magnesium silicate, magnesium aluminate, synthetic hydrotalcite [Mg₆Al₂(OH)₁₆·CO₃·4H₂O], alumina magnesium hydroxide, and the like. Preferred are heavy magnesium carbonate, magnesium carbonate, magnesium oxide, magnesium hydroxide, and the like. Examples of basic inorganic salts of calcium include precipitated calcium carbonate, calcium hydroxide, and the like.

Examples of the "solvent" include water for injection, alcohol, propylene glycol, macrogol, sesame oil, corn oil, olive oil and the like.

Examples of the "solubilizing agent" include polyethylene glycol, propylene glycol, D-mannitol, benzyl benzoate, ethanol, trisaminomethane, cholesterol, triethanolamine, sodium carbonate, sodium citrate and the like.

Examples of the "suspending agent" include surfactants such as stearyltriethanolamine, sodium lauryl sulfate, laurylaminopropionic acid, lecithin, benzalkonium chloride, benzethonium chloride and monostearic glycerol, for example, hydrophilic polymers such as polyvinyl alcohol, polyvinylpyrrolidone, carboxymethyl cellulose sodium, methyl cellulose, hydroxymethyl cellulose, hydroxyethyl cellulose and hydroxypropyl cellulose, and the like.

Examples of the "isotonicity agent" include glucose, D-sorbitol, sodium chloride, glycerin, D-mannitol and the like.

Examples of the "buffering agent" include buffer solutions of phosphate, acetate, carbonate, citrate, etc., and the like.

Examples of the "soothing agent" include benzyl alcohol and the like.

Examples of the "preservative" include p-hydroxybenzoic acid esters, chlorobutanol, benzyl alcohol, phenethyl alcohol, dehydroacetic acid, sorbic acid and the like.

Examples of the "antioxidant" include sulfite, ascorbic acid, α-tocopherol and the like.

Examples of the "coloring agent" include food dyes such as food yellow No. 5, food red No. 2, food blue No. 2, etc.; edible lake dyes, iron oxide red, and the like.

Examples of the "sweetener" include saccharin sodium, dipotassium glycyrrhizinate, aspartame, stevia, somatin and the like.

Examples of the "acidifier" include citric acid (anhydrous citric acid), tartaric acid, malic acid and the like.

Examples of the "foaming agent" include sodium bicarbonate and the like.

The "flavor" may be a synthetic substance or a naturally occurring substance, and examples thereof include lemon, lime, orange, menthol, strawberry and the like.

The crystal of the present invention can be formulated into an oral preparation according to a per se known method, for example, by adding carriers such as an excipient, a disintegrant, a binder and a lubricant to the crystal of the present invention, compression molding the mixture, and then, if necessary, coating the resultant product by a per se known method for the purpose of masking of taste, enteric coating or sustained release. For an enteric-coated preparation, an intermediate layer may be provided between the enteric-coating layer and the drug-containing layer by a per se known method, for the purpose of separation of the two layers.

In the case where the crystal of the present invention is formulated into an orally disintegrating tablet, for example, the orally disintegrating tablet can be produced by coating a core containing crystalline cellulose and lactose with the compound of the present invention and, if necessary, a basic inorganic salt, and then with a coating layer containing a water-soluble polymer to obtain a composition, coating the thus-obtained composition with an enteric coating layer containing polyethylene glycol, then with an enteric coating layer containing triethyl citrate, further with an enteric coating layer containing polyethylene glycol, and finally with mannitol to obtain fine granules, mixing the thus-obtained fine granules with an additive and then molding the mixture.

Examples of the above-described "enteric coating layer" include layers consisting of one or more of an aqueous-type enteric high molecular base such as cellulose acetate phthalate (CAP), hydroxypropylmethyl cellulose phthalate, hydroxymethyl cellulose acetate succinate, a methacrylate copolymer [e.g., Eudragit L30D-55 (trade name; manufactured by Rohm Company), Kollicoat MAE30DP (trade name; manufactured by BASF AG), Poliquid PA30 (trade name; manufactured by Sanyo Kasei Company), etc.], carboxymethylethyl cellulose, shellac or the like; a sustained-release base such as a methacrylate copolymer [e.g., Eudragit NE30D (trade name), Eudragit RL30D (trade name), Eudragit RS30D (trade name), etc.] or the like; a water-soluble high molecular substance; a plasticizer such as triethyl citrate, polyethylene glycol, acetylated monoglyceride, triacetin, castor oil, etc., and the like.

Examples of the above-described "additive" include a water-soluble sugar alcohol (e.g., sorbitol, mannitol, maltitol, reducing saccharized starch, xylitol, reducing palatinose, erythritol, etc.), crystalline cellulose [e.g., Ceolus KG 801, Avicel PH 101, Avicel PH 102, Avicel PH 301, Avicel PH 302, Avicel RC-591 (crystalline cellulose/carmellose sodium), etc.], a low substitution degree hydroxypropyl cellulose [e.g., LH-22, LH-32, LH-23, LH-33 (Shin-Etsu Kagaku Kabushiki Kaisha), a mixture thereof, etc.] and the like. Further, a binder, an acidifier, a foaming agent, a sweetener, a flavor, a lubricant, a coloring agent, a stabilizer, an excipient, a disintegrant and the like may be used.

The crystal of the present invention may be used in combination with one or more, for example, 1 to 3 kinds of other active ingredients.

Examples of said "other active ingredient" include an anti-Helicobacter pylori substance, an imidazole compound, a bismuth salt, a quinolone compound and the like.

Examples of the "anti-Helicobacter pylori substance" include a penicillin antibiotic (e.g., amoxicillin, benzyl penicillin, piperacillin, mecillinam, etc.), a cephem antibiotic (e.g., cefixime, cefaclor, etc.), a macrolide antibiotic (e.g., erythromycin, clarithromycin, etc.), a tetracycline antibiotic (e.g., tetracycline, minocycline, streptomycin, etc.), an aminoglycoside antibiotic (e.g., gentamycin, amikacin, etc.), imipenem and the like. Among them, preferred are a penicillin antibiotic, a macrolide antibiotic and the like.

Examples of the "imidazole compound" include metronidazole, miconazole and the like.

Examples of the "bismuth salt" include bismuth acetate, bismuth citrate and the like.

Examples of the "quinolone compound" include ofloxacin, ciproxacin and the like.

In particular, for the purpose of anti-Helicobacter pylori (including bacteria elimination and assistance of bacteria elimination), the crystal of the present invention is preferably used in combination with a penicillin antibiotic (e.g., amoxicillin) and an erythromycin antibiotic (e.g., clarithromycin).

The "other active ingredient" and the crystal of the present invention may be mixed and formulated into a single pharmaceutical composition [e.g., a tablet, a powder, a granule, a capsule (including a soft capsule), a liquid, an injectable preparation, a suppository, a sustained-release preparation, etc.] according to a per se known method, or may be formulated separately and administered to the same subject at the same time or at a certain interval.

In the case where the crystal of the present invention is formulated into, for example, an injectable preparation, the injectable preparation can be usually dissolved in or diluted with a solvent which is substantially free from any nonaqueous solvent (or any water-soluble organic solvent) and whose medium is substantially water. If necessary, a strong alkali or a chelating agent may be added to the resulting solution or dilution.

The injectable preparation is diluted until the pH of the resulting dilution reaches about 9 to 12, prior to use. When the crystal of the present invention in an amount corresponding to 30 mg of 2-[[[3-methyl-4-(2,2,2-trifluoroethoxy)-2-pyridinyl]methyl]sulfinyl]-1H-benzimidazole (hereinafter, sometimes, referred to as the physiologically active ingredient) is dissolved in 5 ml of physiological saline or distilled water for injection, it is preferable that the pH of the solution reaches about 9 to 12, more preferably about 10.4 to about 12.0.

The injectable preparation may further contain N-methylglucamine, for the purpose of suppressing the lowering of pH and stabilizing the solubility when an injectable solution is prepared, or the like. The amount of N-methylglucamine to be added may be about 0.1 mg to about 1 mg per 1 mg of the physiologically active ingredient. The injectable preparation may further contain a saccharide (e.g. a sugar alcohol such as mannitol, etc.) for the purpose of stabilization of a shape in the case of a solid injectable preparation, or the like. The amount of a saccharide to be added may be about 0.1 mg to about 20 mg per 1 mg of the physiologically active ingredient. An example of the injectable preparation containing such ingredients is an injectable preparation containing the physiologically active ingredient and capable of being dissolved in or diluted with a solvent substantially free from a nonaqueous solvent, which may contain about 0.1 mg to about 0.8 mg of N-methylglucamine and about 1 mg to about 10 mg of a sugar alcohol per 1 mg of the physiologically active ingredient.

When the physiologically active ingredient is used in combination with a chelating agent, the chelating agent may be previously mixed with the physiologically active ingredient and, if necessary, other ingredients to be formulated into a preparation and then provided. Alternatively, the chelating agent may be kept separately from a preparation containing the physiologically active ingredient, and these may be mixed just prior to use to obtain an injectable preparation. Examples of the chelating agent include edetic acid or a salt or a derivative thereof, phosphoric acid or a salt thereof, citric acid or a salt thereof, and the like. These chelating agents may be used alone or as a mixture of two or more kinds. Particularly preferred is edetic acid or a salt thereof. For example, an injectable preparation containing edetic acid or a salt thereof in an amount of about 0.03% to about 67%, preferably about 0.3% to about 33%, more preferably about 0.6% to about 6.7% of the weight of the physiologically active ingredient does not form particulate insolubles when the injectable prepration is filled into a plastic container, thereby a high-quality injectable preparation can be provided. As a salt of edetic acid, a sodium salt, a calcium salt, a mixture of them, or the like is preferably used. That is, preferable examples of a salt of edetic acid include a sodium salt, a calcium salt of edetic acid, a sodium and calcium salt of edetic acid (calcium disodium edetate, etc.) and the like. Particularly preferred is a sodium salt of edetic acid, such as disodium edetate, tetrasodium edetate or calcium disodium edetate. Especially preferred is disodium edetate. Edetic acid or a salt thereof may be usually used in an amount of about 0.03% to about 67% of the weight of the physiologically active ingredient.

The injectable preparation preferably contains about 0.009 mg to about 20.1 mg of a single chelating agent such as disodium edetate, tetrasodium edetate or calcium disodium edetate, or a combination of chelating agents, about 8 mg to about 24 mg of N-methylglucamine, and about 50 mg to about 70 mg of mannitol, per 30 mg of the physiologically active ingredient. The injectable preparation of the present invention may be a lyophilized preparation (a lyophilized injectable preparation). The chelating agent such as disodium edetate, tetrasodium edetate or calcium disodium edetate may be packed in a container separate from a container containing the injectable preparation comprising the other ingredients.

The lyophilized injectable preparation can be dissolved in at least one liquid or solvent selected from water for injection (distilled water for injection), an infusion solution containing an electrolytic solution (e.g. physiological saline, etc.) and the like, a nutrient infusion and the like, thereby an injectable solution can be easily prepared. The injectable solution can be packed in a glass container or a plastic container.

The term "injectable preparation" as used herein means not only an injectable solution as the final form, but also a precursor of an injectable solution from which the final injectable solution can be prepared using a solvent prior to use [for example, a liquid injectable preparation (e.g. a concentrated or condensed injectable preparation) or a solid injectable preparation (e.g. a lyophilized injectable prepration)].

As a container for the injectable preparation, various containers including a glass container and a plastic container can be used regardless of their materials. Examples of a plastic container include containers of polyethylene, polypropylene, a polyethylene-polypropylene copolymer, polyvinyl chloride, an ethylene-vinyl acetate copolymer, an ethylene-propylene copolymer, silicone, polybutadiene, a thermoplastic elastomer, Teflon (Registered Trade Mark), polyurethane, cyclic polyolefin, and polyolefin.

The following Reference Examples and Examples further illustrate the present invention in more detail, but they are not intended to limit the present invention.

In the following Reference Examples and Examples, room temperature means about 15 to 30°C.

Elemental analysis was carried out with Vario EL for C, H and N and with ICS-1500 for S and F.

Melting points were measured with BUCHI Melting Point B-540, and uncorrected numerical values are shown.

IR spectra were measured with FT-IR Thermoelectron Nicolet 4700.

X-ray powder diffraction was measured with X-ray Powder Diffractometer Rigaku RINT Ultima+.

Atomic absorption (Na) was measured by Shimadzu AA-6300 atomic absorption photometer.

¹H-NMR spectra was measured with BURKER DPX300 using DMSO-d₆ as a solvent, and chemical shifts δ (ppm) from tetramethylsilane used as the internal standard are shown.

Meanings of the other symbols used herein are as follows:
s : singlet
d : doublet
m : multiplet
J : a coupling constant

### Reference Example 1

### Synthesis of 2-[[[3-methyl-4-(2,2,2-trifluoroethoxy)-2-pyridinyl]methyl]sulfinyl]-1H-benzimidazole (lansoprazole) sodium salt (amorphous)

2-[[[3-Methyl-4-(2,2,2-trifluoroethoxy)-2-pyridinyl]methyl]sulfinyl]-1H-benzimidazole (lansoprazole) (110.8 g) was dissolved in a mixture of ethanol (400 mL), a 1 N aqueous sodium hydroxide solution (315 mL) and water (50 mL) at room temperature, and then concentrated to dryness. The residue was dried overnight under reduced pressure at room temperature to give a sodium salt of lansoprazole (119 g) as an amorphous substance.

X-ray powder diffraction: No particular peak was observed.

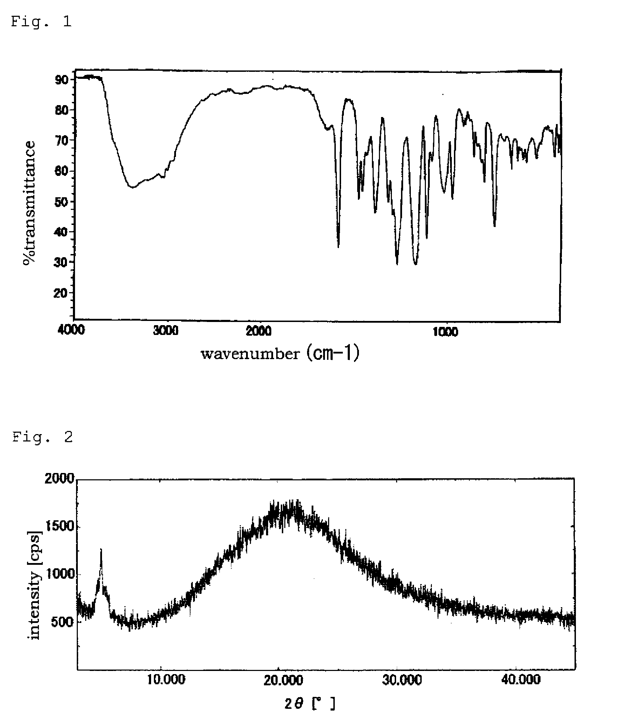

An example of an IR chart and an example of an X-ray powder diffraction chart of the resulting lansoprazole sodium salt (amorphous) are shown in Fig. 1 and Fig. 2 respectively.

### Example 1

### Synthesis of a crystal of a sodium salt of 2-[[[3-methyl-4-(2,2,2-trifluoroethoxy)-2-pyridinyl]methyl]sulfinyl]-1H-benzimidazole (lansoprazole)

The amorphous lansoprazole sodium salt (20 g) obtained in Reference Example 1 was suspended in acetone (200 mL) at room temperature, heated to 40°C and stirred. The suspension was gradually cooled to room temperature and then stirred overnight. Then, the suspension was cooled on ice and filtered to collect crystals. The crystals obtained were dried under reduced pressure at 40°C to give 10.3 g of a sodium salt of lansoprazole as crystals.
Elementary analysis:
Calcd.: C: 49.11, H: 3.35, N: 10.74, S: 8.19, F: 14.56, O: 818, Na: 5.87
Found: C: 48.90, H: 3.33, N: 10.70, S: 8.05, F: 14.65
Melting point: 251°C (decomposed)
IR (ν cm⁻¹) : 1584, 1266, 1182, 1025, 742.
X-ray powder diffraction (2θ°) : 5.64, 14.24, 17.18, 19.72, 20.50, 20.96, 21.40, 29.14°
Atomic absorption (Na): 6.1% (calculated value: 5.9%)
¹H-NMR: 2.26 (3H, s), 4.42 (1H, d, J=12.8 Hz), 4.84-4.93 (4H, m), 6.83-6.88 (2H, m), 7.05 (1H, d, J=5.7 Hz), 7.41-7.46 (2H, m), 8.35 (1H, d, J=5.6 Hz).

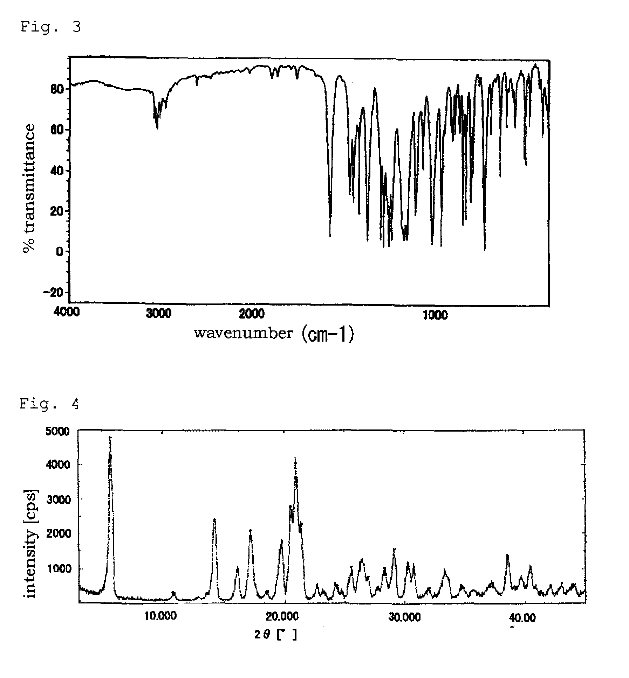

An example of an IR chart and an example of an X-ray powder diffraction chart of the resulting lansoprazole sodium salt crystal are shown in Fig. 3 and Fig. 4 respectively.

### Preparation Reference Example 1

### Production of capsules containing crystals of 2-[[[3-methyl-4-(2,2,2-trifluoroethoxy)-2-pyridinyl]methyl]-sulfinyl]-1H-benzimidazole (lansoprazole) sodium salt

Capsules of the formulation shown in Table 3 are produced using the feeding amount shown in Table 1 or 2 by the following method.

2-[[[3-Methyl-4-(2,2,2-trifluoroethoxy)-2-pyridinyl]methyl]sulfinyl]-1H-benzimidazole (lansoprazole) sodium salt crystals (1) and ingredients (3) to (6) are well mixed to prepare a dusting powder. Nonpareils (2) are put into a centrifugal fluidized coating granulator, and then coated with the dusting powder while being sprayed with an aqueous solution of hydroxypropyl cellulose (7) in purified water, to give spherical granules. The spherical granules are dried in vacuum at 40°C for 16 to 18 hours and then passed through a sieve (500 µm, 1190 µm) to give base-drug granules. Two batches of the base-drug granules are put into a flow coater and coated with a suspension of the ingredients from methacrylic acid copolymer LD (8) to polysorbate 80 (12) in purified water. Talc (13) is added to the coated granules, which are passed through a sieve (600 µm, 1420 µm) and dried in vacuum at 42°C for 16 to 18 hours to give enteric-coated granules. Talc (14) and light silicic anhydride (15) are added to 1 batch of the enteric-coated granules (given the feeding amounts shown in Tables 2 and 3, the enteric-coated granules can be mixed in amounts of up to 5 and 3 batches, respectively) and mixed in a tumbler mixer to give mixed granules. The mixed granules are filled into a gelatin capsule No. 1 (16) and a gelatin capsule No. 3 (17) using a capsule filler to give a 30-mg capsule and a 15-mg capsule, respectively.

**[Table 1]**

| Feeding amount -1 | | | | | |
|---|---|---|---|---|---|
| Composition | | | | Common among 15-mg and 30-mg capsules | |
| [Base-drug granule] | | | | | |
| (1) Compound A*¹ | | | | 4.481 kg *2 | |
| (2) Sucrose-starch spherical granule (nonpareil) | | | | 15.950 kg | |
| (3) Magnesium carbonate | | | | 3.345 kg *2 | |
| (4) Refined sucrose | | | | 8.931 kg *2 | |
| (5) Corn starch | | | | 5.436 kg *2 | |
| (6) Low-substituted hydroxypropyl cellulose | | | | 5.974 kg *2 | |
| (7) Hydroxypropyl cellulose | | | | 0.203 kg | |
| Purified water | | | | (10.297 L) | |
| Subtotal | | | | 43.500 kg | |

| [Enteric-coated granule] | | | | | |
|---|---|---|---|---|---|
| Base-drug granule | | | | 87.000 kg | |
| (8) Methacrylic acid copolymer LD | | | | 13.5807 kg *3, *5 | |
| (9) Talc | | | | 4.0803 kg *4 | |
| (10) Macrogol 6000 | | | | 1.3398 kg *4 | |
| (11) Titanium oxide | | | | 1.3398 kg *4 | |
| (12) Polysorbate 80 | | | | 0.690 kg *4 | |
| Purified water | | | | (95.004L) *4 | |
| (13) Talc | | | | 0.116 kg | |
| Subtotal | | | | 107.068 kg | |

| [Mixed granule]*5 | | | | | |
|---|---|---|---|---|---|
| Enteric-coated granule | 107.068 kg | 214.136 kg | 321.204 kg | 428.272 kg | 535.340 kg |
| (14) Talc | 0.058 kg | 0.116 kg | 0.174 kg | 0.232 kg | 0.290 kg |
| (15) Light silicic anhydride | 0.174 kg | 0.348 kg | 0.522 kg | 0.696 kg | 0.870 kg |
| Subtotal | 107.300 kg | 214.600 kg | 321.900 kg | 429.200 kg | 536.500 kg |

| [Capsule] | | | | | |
|---|---|---|---|---|---|
| Mixed granule | 107.300 kg | 214.600 kg | 321.900 kg | 429.200 kg | 536.500 kg |
| (16)Gelatin Capsule No. 1 *6 | 290,000 | 580,000 | 870,000 | 1,180,000 | 1,450,000 |
| (17)Gelatin Capsule No. 3 *7 | 580,000 | 1,160,000 | 1,740,000 | 2,320,000 | 2,900,000 |

| | | | | | |
|---|---|---|---|---|---|
| *1: 2-[[[3-methyl-4-(2,2,2-trifluoroethoxy)-2-pyridinyl]-methyl]sulfinyl]-1H-benzimidazole (lansoprazole) sodium salt crystal *2: Fed in an amount increased by 3% *3: Solid content *4: Fed in an amount increased by 5% *5: 1 to 5 batches of enteric-coated granules can be mixed *6: Number of capsules in the case of production of a 30-mg capsule *7: Number of capsules in the case of production of a 15-mg capsule | | | | | |

**[Table 2]**

| Feeding amount -2 | | | |
|---|---|---|---|
| Composition | | Common among 15-mg and 30-mg capsules | |
| [Base-drug granule] | | | |
| (1) Compound A*¹ | | 8.953 kg *2 | |
| (2) Sucrose-starch spherical granule (nonpareil) | | 24.750 kg | |
| (3) Magnesium carbonate | | 5.191 kg *2 | |
| (4) Refined sucrose | | 13.860 kg *2 | |
| (5) Corn starch | | 8.436 kg *2 | |
| (6) Low-substituted hydroxypropyl cellulose | | 9.270 kg *2 | |
| (7) Hydroxypropyl cellulose | | 0.315 kg | |
| Purified water | | (15.435 L) | |
| Subtotal | | 67.500 kg | |

| [Enteric-coated granule] | | | |
|---|---|---|---|
| Base-drug granule | | 135.000 kg | |
| (8) Methacrylic acid copolymer LD | | 21.0757 kg *3, *5 | |
| (9) Talc | | 6.832 kg *4 | |
| (10) Macrogol 6000 | | 2.079 kg *4 | |
| (11) Titanium oxide | | 2.079 kg *4 | |
| (12) Polysorbate 80 | | 0.945 kg *4 | |
| Purified water | | (98.910 L) *4 | |
| (13) Talc | | 0.180 kg | |
| Subtotal | | 166.140 kg | |

| [Mixed granule] *5 | | | |
|---|---|---|---|
| Enteric-coated granule | 166.140 kg | 332.280 kg | 498.420 kg |
| (14) Talc | 0.090 kg | 0.180 kg | 0.270 kg |
| (15) Light silicic anhydride | 0.270 kg | 0.540 kg | 0.810 kg |
| Subtotal | 166.500 kg | 333.000 kg | 499.500 kg |

| [Capsule] | | | |
|---|---|---|---|
| Mixed granule | 166.500 kg | 333.000 kg | 499.500 kg |
| (16) Gelatin Capsule No. 1 *6 | 450,000 | 900,000 | 1,350,000 |
| (17) Gelatin Capsule No. 3 *7 | 900,000 | 1,800,000 | 2,700,000 |

| | | | |
|---|---|---|---|
| *1: 2-[[[3-methyl-4-(2,2,2-trifluoroethoxy)-2-pyridinyl]-methyl]sulfinyl]-1H-benzimidazole (lansoprazole) sodium salt crystal *2: Fed in an amount increased by 3% *3: Solid content *4: Fed in an amount increased by 5% *5: 1 to 3 batches of enteric-coated granules can be mixed *6: Number of capsules in the case of production of a 30-mg capsule *7: Number of capsules in the case of production of a 15-mg capsule | | | |

**[Table 3]**

| Formulation per capsule | | |
|---|---|---|
| Composition | 15 mg capsule | 30 mg capsule |
| [Base-drug granule] | | |
| (1) Compound A*¹ | 15.0 mg | 30.0 mg |
| (2) Sucrose-starch spherical granule (nonpareil) | 55.0 mg | 110.0 mg |
| (3) Magnesium carbonate | 11.2 mg | 22.4 mg |
| (4) Refined sucrose | 29.9 mg | 59.8 mg |
| (5) Corn starch | 18.2 mg | 36.4 mg |
| (6) Low-substituted hydroxypropyl cellulose | 20.0 mg | 40.0 mg |
| (7) Hydroxypropyl cellulose | 0.7 mg | 1.4 mg |
| Subtotal | 150.0 mg | 300.0 mg |

| [Enteric-coated granule] | | |
|---|---|---|
| Base-drug granule | 150.0 mg | 300.0 mg |
| (8) Methacrylic acid copolymer LD | 22.3 mg | 44.8 mg |
| (9) Talc | 6.7 mg | 13.4 mg |
| (10) Macrogol 6000 | 2.2 mg | 4.4 mg |
| (11) Titanium oxide | 2.2 mg | 4.4 mg |
| (12) Polysorbate 80 | 1.0 mg | 2.0 mg |
| (13) Talc | 0.2 mg | 0.4 mg |
| Subtotal | 184.6 mg | 369.2 mg |

| [Mixed granule] *5 | | |
|---|---|---|
| Enteric-coated granule | 184.6 mg | 369.2 mg |
| (14) Talc | 0.1 mg | 0.2 mg |
| (15) Light silicic anhydride | 0.3 mg | 0.6 mg |
| Subtotal | 185.0 mg | 370.0 mg |

| [Capsule] | | |
|---|---|---|
| Mixed granule | 185.0 mg | 370.0 mg |
| (16) Gelatin Capsule No. 1 | - | 79.0 mg |
| (17) Gelatin Capsule No. 3 | 50.0 mg | - |
| Subtotal | 235.0 mg | 449.0 mg |

| | | |
|---|---|---|
| *1: 2-[[[3-methyl-4-(2,2,2-trifluoroethoxy)-2-pyridinyl]-methyl]sulfinyl]-1H-benzimidazole (lansoprazole) sodium salt crystal | | |

### Industrial Applicability

The 2-[[[3-methyl-4-(2,2,2-trifluoroethoxy)-2-pyridinyl]methyl]sulfinyl]-1H-benzimidazole salt crystal of the present invention has low toxicity and has an excellent antiulcer activity, gastric acid antisecretory activity, mucosal protective activity, anti-Helicobacter pylori activity and the like, and therefore it is useful as a medicine.

## Claims

1. A crystal of a sodium salt of 2-[[[3-methyl-4-(2,2,2-trifluoroethoxy)-2-pyridinyl]methyl]sulfinyl]-1H-benzimidazole.

2. The crystal according to claim 1, which has infrared absorption spectra at wavenumbers of about 1584, 1266, 1182, 1025 and 742 cm⁻¹.

3. The crystal according to claim 1, which has an X-ray powder diffraction pattern whose characteristic peaks appear at about 5.64, 14.24, and 20.96° that are diffraction angles represented by 2θ in X-ray powder diffraction.

4. A pharmaceutical composition comprising the crystal according to claim 1.

5. The pharmaceutical composition according to claim 4, which is a prophylactic or remedy for peptic ulcer, Zollinger-Ellison syndrome, gastritis, reflux esophagitis, symptomatic GERD, NUD (Non-Ulcer Dyspepsia), gastric cancer, gastric MALT lymphoma or hyperacidity, an anti-Helicobacter pylori agent, or an inhibitor for upper gastrointestinal hemorrhage due to peptic ulcer, acute stress ulcer, hemorrhagic gastritis or invasive stress.

6. A method for preventing or treating peptic ulcer, Zollinger-Ellison syndrome, gastritis, reflux esophagitis, symptomatic GERD, NUD, gastric cancer, gastric MALT lymphoma or hyperacidity, eliminating Helicobacter pylori, or inhibiting upper gastrointestinal hemorrhage due to peptic ulcer, acute stress ulcer, hemorrhagic gastritis or invasive stress, which comprises administering an effective amount of the crystal of claim 1 to a mammal.

7. Use of the crystal according to claim 1 for production of a prophylactic or remedy for peptic ulcer, Zollinger-Ellison syndrome, gastritis, reflux esophagitis, symptomatic GERD, NUD, gastric cancer, gastric MALT lymphoma or hyperacidity, an anti-Helicobacter pylori agent, or an inhibitor for upper gastrointestinal hemorrhage due to peptic ulcer, acute stress ulcer, hemorrhagic gastritis or invasive stress.
